# EUROPEAN PATENT APPLICATION

(11) **EP 4 721 798 A1**
(43) Date of publication of application: **08.04.2026**
(21) Application number: 23938954.7
(22) Date of filing: 01.06.2023
(51) Int. Cl.: A61M 16/20, A61M 16/01

(54) **SWITCHING APPARATUS AND VENTILATION DEVICE**

(71) Applicant: Shenzhen Mindray Bio-Medical Electronics Co., Ltd., Shenzhen, Guangdong 518057 (CN)
(72) Inventor: LUO, Caijin, Shenzhen, Guangdong 518057 (CN); WU, Xuetao, Shenzhen, Guangdong 518057 (CN)
(74) Representative: KIPA AB
(86) International application number: PCT/CN2023/097822
(87) International publication number: WO 2024/243967

(57) **Abstract**

A switching apparatus and a ventilation device. The switching apparatus comprises a handle assembly (100) and a valve assembly (200); the valve assembly (200) comprises a valve body (201) and a valve core (202); the valve body (201) is provided with a valve cavity (203), a breathing circuit interface (204), a machine-controlled branch interface (205), and a manual branch interface (206); the handle assembly (100) comprises a support (20) fixed to a ventilation device, a handle body (10) rotatably connected to the support (20), and an output member for driving the valve assembly (200); the handle body (10) can rotate relative to the support (20); the output member is pushed against the valve core (202), and moves between a first position (311) and a second position (312) during the rotation of the handle body (10), so as to drive the valve core (202) to perform reciprocating motion in a first direction, such that the valve assembly (200) is switched between a first mode and a second mode; and the handle assembly (100) and the valve assembly (200) are separately designed, and a force is applied to the valve assembly (200) in at least one of the first mode and the second mode, such that when the support (20) is fixed to the ventilation device, the valve assembly (200) is separated from or coupled with the handle assembly (100), so as to disassemble or assemble the valve assembly (200).

## Description

### TECHNICAL FIELD

The disclosure relates to a technical field of medical device, and more particularly to a switching apparatus and a ventilation device.

### BACKGROUND

Anesthesia machine is a common medical anesthesia device, which delivers anesthetic into alveoli of a patient through a respiratory loop to form a partial pressure of an anesthetic gas, and directly inhibits a central nervous system after diffusing the anesthetic into blood, thereby producing an effect of general anesthesia.

Wherein, the anesthesia machine is switched between manual ventilation and machine-controlled ventilation through a switching apparatus, which is usually provided with a handle unit and a valve unit that are interconnected with each other. The valve unit is assembled into a respiratory loop and is in contact with a patient gas, and needs to be disassembled for sterilization. During sterilization, the handle unit and the valve unit are dissembled together, which brings a lot of inconvenience.

### SUMMARY

This disclosure provides a switching apparatus and a ventilation device, which is capable of more conveniently sterilizing a valve unit.

According to a first aspect, an embodiment of this disclosure provides a switching apparatus for a ventilation device, including a handle unit and a valve unit, wherein the valve unit includes a valve body and a valve core, wherein the valve body is provided with a valve cavity, a respiratory loop interface, a machine-controlled branch interface and a manual branch interface;
the handle unit includes a support which is capable of being fixed to the ventilation device, a handle body which is rotatably connected with the support, and an output member which is configured to drive the valve unit; wherein the handle body is capable of rotating relative to the support, the output member abuts against the valve core and moves between a first position and a second position while the handle body rotates, so as to drive the valve core to reciprocate along a first direction, and enable the valve unit to be switched between a first mode and a second mode; wherein in the first mode, the respiratory loop interface is connected with the manual branch interface; in the second mode, the respiratory loop interface is connected with the machine-controlled branch interface;
wherein the handle unit and the valve unit are designed as separate units, and when the support is fixed to the ventilation device and a force is applied to the valve unit in at least one of the first mode or the second mode, the valve unit is separated from or coupled with the handle unit, so as to disassemble or assemble the valve unit.

In a switching apparatus according to an embodiment of this disclosure, a first elastic member is arranged between the valve core and the valve body, wherein the output member and the first elastic member apply forces in opposite directions on the valve core along the first direction.

In a switching apparatus according to an embodiment of this disclosure, the output member moves along the first direction; the first position and the second position have a height difference along the first direction; the output member is located outside the valve cavity while the output member moves from the first position to the second position.

In the switching apparatus according to an embodiment of this disclosure, the valve body is provided with an abutting surface, the valve core is assembled inside the valve cavity and at least extends from the abutting surface in said at least one of the first mode or the second mode.

In a switching apparatus according to an embodiment of this disclosure, at least one of the valve core and the handle unit is arranged with a guide structure, which guides the valve unit while assembling the valve unit.

In a switching apparatus of one embodiment of this disclosure, the guide structure includes a first guide structure and a second guide structure, the first guide structure is arranged at the valve core, the second guide structure is arranged at the handle unit, the first guide structure fits with the second guide structure; wherein, the first guide structure is a first inclined guide surface arranged at the valve core, and the second guide structure is a second inclined guide surface arranged at the handle unit.

In a switching apparatus according to an embodiment of this disclosure, the valve core includes a guide seat and a valve rod, wherein the guide seat is assembled at one end of the valve rod which end is adjacent to the handle unit.

In a switching apparatus according to an embodiment of this disclosure, a recessed portion is arranged at the valve body; a first assembling portion which extends along the first direction and a first through-hole which penetrates through the first assembling portion are arranged inside the recessed portion; wherein the guide seat is assembled at the first assembling portion and is capable of moving along the first direction, and the valve rod penetrates through and is arranged inside the first through-hole.

In the switching apparatus according to an embodiment of this disclosure, a guide structure is arranged between the guide seat and the recessed portion, or between the guide seat and the first assembling portion.

In the switching apparatus according to an embodiment of this disclosure, the valve core includes a first sealing member, which is arranged between an inner wall of the first through-hole and the valve rod.

In a switching apparatus of one embodiment of this disclosure, the valve core includes a diaphragm, which is fixed at the valve rod and is capable of moving along the first direction with the valve rod, so as to connect the respiratory loop interface and the machine-controlled branch interface, or so as to connect the respiratory loop interface and the manual branch interface.

In a switching apparatus according to an embodiment of this disclosure, the output member includes a driving member and a fitting member for guide groove, wherein a guide groove is arranged at the handle body, one end of the driving member is in transmission fitting with the guide groove through the fitting member for guide groove, and the other end of the driving member abuts against the valve core.

In a switching apparatus of one embodiment of this disclosure, the fitting member for guide groove is arranged inside the guide groove and fits with the guide groove; wherein one end of the fitting member for guide groove is connected with the driving member; an anti-rotation groove which extends along the first direction is arranged at the support, and the other end of the fitting member for guide groove is movably connected with the anti-rotation groove.

In a switching apparatus of one embodiment of this disclosure, the fitting member for guide groove is sphere-shaped or arc-shaped, and an elongated groove is arranged inside the guide groove along an extension direction of the guide groove; wherein one end of the driving member penetrates through the elongated groove and is connected with the fitting member for guide groove, or one end of the driving member is connected with the fitting member for guide groove through a linking member which penetrates through the elongated groove.

In a switching apparatus according to an embodiment of this disclosure, the guide groove includes a first track groove and a second track groove that are connected with each other, wherein the first track groove intersects with the second track groove to form an obtuse angle.

In the switching apparatus according to an embodiment of this disclosure, at least one of the first track groove and the second track groove is inclined from an intersection of the first track groove and the second track groove toward a direction which is away from the valve body.

In a switching apparatus according to an embodiment of this disclosure, one end of the fitting member for guide groove is connected with the driving member, and the fitting member for guide groove is arranged inside the guide groove; or
the output member further includes a rolling member, one end of the fitting member for guide groove is connected with the driving member, and the fitting member for guide groove is movably assembled inside the guide groove through the rolling member.

In a switching apparatus of one embodiment of this disclosure, a first U-shaped structure is formed at a lower end of the output member, and an opening direction of the first U-shaped structure is toward a disassembling direction of the valve unit; a second U-shaped structure with an opposite opening direction is formed at an upper end of the valve rod, wherein the first U-shaped structure fits with the second U-shaped structure and abuts against the second U-shaped structure along the first direction.

In a switching apparatus according to an embodiment of this disclosure, the handle unit includes a pre-tightened member which is assembled at the support and abuts against the handle body, so as to prevent the handle body from rotating relative to the support when the output member is located at the first position and the second position.

In a switching apparatus of one embodiment of this disclosure, the pre-tightened member includes a second elastic member and a pre-tightened wheel which wheel fits with the handle body, wherein one end of the second elastic member abuts against the support, and the other end of the second elastic member abuts against the pre-tightened wheel, so as to enable the pre-tightened wheel to abut against the handle body.

In a switching apparatus of one embodiment of this disclosure, a protruded structure is arranged at one of the pre-tightened wheel and the handle body; and a recessed structure is arranged at the other of the pre-tightened wheel and the handle body, wherein the recessed structure is provided with a recessed inclined surface; when the output member is located at the first position and the second position, the protruded structure is snap-fitted into the recessed structure under an action of the second elastic member; when the handle body rotates around the support, the protruded structure abuts against the recessed inclined surface and presses the second elastic member as a height of the recessed inclined surface increases.

In the switching apparatus according to an embodiment of this disclosure, the handle unit includes a first travel switch which is configured to detect a position of the handle body, wherein the first travel switch is assembled at the support.

In the switching apparatus according to an embodiment of this disclosure, the first travel switch is provided with a first triggering portion, which extends toward one side of the handle body.

In the switching apparatus according to an embodiment of this disclosure, the handle unit includes a second travel switch which is configured to detect a position of the valve core of the valve unit, wherein the second travel switch is assembled at the support.

In the switching apparatus according to an embodiment of this disclosure, the second travel switch is provided with a second triggering portion, which extends toward one end of the valve core.

According to a second aspect of this disclosure, this disclosure further provides a ventilation device, including a respiratory loop and the above-mentioned switching apparatus, wherein the switching apparatus is assembled inside the respiratory loop, and the valve unit is connected with the respiratory loop.

In a ventilation device according to an embodiment of this disclosure, the ventilation device includes a main body of ventilation device, a first snap-fit structure which is arranged at the valve unit, and a second snap-fit structure which is arranged at the main body of ventilation device; when the valve unit is separated from or coupled with the handle unit, the first snap-fit structure and the second snap-fit structure are correspondingly separated from or snap-fitted with each other.

In a ventilation device according to an embodiment of this disclosure, a third guide structure is arranged at the valve unit, and a fourth guide structure is arranged at the main body; wherein the third guide structure moves along the fourth guide structure, while the valve unit and the handle unit are separated from or coupled with each other.

In the ventilation device according to an embodiment of this disclosure, the third guide structure is a slide block which is arranged at the valve unit, and the fourth guide structure is a slide track which is arranged at the ventilation device.

The technical solution provided by the embodiment of this disclosure may include following beneficial effects. This disclosure designs a switching apparatus and a ventilation device, the switching apparatus includes a handle unit and a valve unit, wherein the valve unit is provided with a valve cavity, a respiratory loop interface, a machine-controlled branch interface and a manual branch interface, wherein the respiratory loop interface is connected with the respiratory loop, the machine-controlled branch interface is connected with the machine-controlled branch, and the manual branch interface is connected with the manual branch. The handle unit is configured to control the valve unit to switch the respiratory loop interface to connect the machine-controlled branch interface or the manual branch interface. Since the handle unit and the valve unit are designed as separate units, the handle unit is located outside the valve cavity of the valve unit, so as to isolate the handle unit from a respiratory gas or an anesthetic gas of patient, and only allow the valve unit to contact the respiratory gas or the anesthetic gas of patient, that is, when the switching apparatus is sterilized, the handle unit does not need to be sterilized, which not only reduces a labor intensity, but also reduces a temperature and chemical resistance requirement of the handle unit, avoiding a possibility of damage to the handle unit during sterilization.

Specifically, the handle unit includes a fixed support and a handle which is rotatably connected with the support, wherein the handle is capable of rotating relative to the support, so as to drive the valve core to move in a first direction, so as to enable the valve unit to be switched from a first mode to a second mode; wherein in the first mode, the respiratory loop interface is connected with the manual branch interface; in the second mode, the respiratory loop interface is connected with the machine-controlled branch interface, thereby realizing a switching of a ventilation mode of the ventilation device with a high reliability, while maintaining an original operation mode which is in line with an operation habit of medical staff.

It should be understood that the above general description and the following detailed description are only exemplary and explanatory, and do not limit the disclosure.

### BRIEF DESCRIPTION OF THE DRAWINGS

In order to more clearly explain the embodiments of this disclosure, the following briefly introduces the drawings which are needed to be used in the description of the embodiments. It is obvious that the drawings in the following description are only some embodiments of this disclosure. For those skilled in the art, other drawings can be obtained from these accompanying drawings without paying any creative works.
FIG. 1 is a cross-sectional diagram of a switching apparatus according to an embodiment of this disclosure.
FIG. 2 is a cross-sectional diagram of the switching apparatus in FIG. 1, in which a handle body is in a second state.
FIG. 3 is a cross-sectional diagram of a handle unit in FIG. 1, in which a handle body is in a second state.
FIG. 4 is a cross-sectional diagram of the handle unit in FIG. 1, in which a handle body is in a first state.
FIG. 5 is a structural diagram of a handle unit in FIG. 1.
FIG. 6 is a structural diagram of a handle unit in FIG. 1 at another angle.
FIG. 7 is a cross-sectional diagram of a handle unit in FIG. 1.
FIG. 8 is an exploded schematic diagram of a handle unit in FIG. 1.
FIG. 9 is a partial structural diagram of a handle unit in FIG. 1.
FIG. 10 is a cross-sectional diagram of a valve unit in FIG. 1 in a first state.
FIG. 11 is a cross-sectional diagram of a valve unit in FIG. 1 in a second state.
FIG. 12 is a partial cross-sectional diagram of a valve unit in FIG. 1.
FIG. 13 is a schematic diagram of a guide groove in FIG. 2, wherein the guide groove is in a line shape.
FIG. 14 is another schematic diagram of a guide groove in FIG. 2, wherein the guide groove is in a bent shape.
FIG. 15 is another schematic diagram of a guide groove in FIG. 2, wherein the guide groove is in a V shape.
FIG. 16 is another schematic diagram of a guide groove in FIG. 2, wherein the guide groove is in an inverted V shape.
FIG. 17 is another schematic diagram of a guide groove in FIG. 2, wherein the guide groove is in an outwardly convex arc shape.
FIG. 18 is another schematic diagram of a guide groove in FIG. 2, wherein the guide groove is in a concave arc shape.
FIG. 19 is a schematic diagram of a switching apparatus according to another embodiment of this disclosure.
FIG. 20 is a schematic diagram of a switching apparatus according to another embodiment of this disclosure.

### Description of reference numerals:

100, handle unit; 10, handle body; 11, operation portion; 12, second inclined guide surface; 13, anti-rotation frame; 131, anti-rotation groove; 132, position-limiting platform; 14, recessed structure; 15, triggering member; 20, support; 21, pre-tightened member groove; 22, position-limiting groove; 23, guide hole; 30, cam structure; 31, guide groove; 311, first extreme position; 312, second extreme position; 40, rotation shaft; 50, fitting member for guide groove; 60, driving member; 70, rolling member; 80, detection unit; 81, first travel switch; 82, second travel switch; 90, pre-tightened member; 91, pre-tightened wheel; 911, protruded structure; 92, second elastic member; 93, assembling member for pre-tightened member; 94, fixation support frame; 200, valve unit; 201, valve body; 2011, abutting surface; 202, valve core; 2021, valve rod; 2022, diaphragm; 2023, guide seat; 20231, first inclined guide surface; 2024, first assembling portion; 2025, recessed portion; 203, valve cavity; 204, gas inlet; 205, first gas outlet; 206, second gas outlet; 207, connection portion of valve rod; 208, assembling portion for diaphragm; 209, first elastic member.

### DESCRIPTION OF THE PREFERRED EMBODIMENTS

The technical solutions in the embodiments of this disclosure are described clearly and completely below in combination with the drawings in the embodiments of this disclosure. Obviously, the described embodiments are just some of the embodiments of this disclosure, not all of them. According to the embodiments in this disclosure, all other embodiments obtained by those skilled in the art without making creative work fall within the protection scope of this disclosure.

It should also be understood that the terms used in this description of this disclosure are only for describing specific entities. In the description of this disclosure, it should be understood that the terms "center", "longitudinal", "lateral", "length", "width", "thickness", "up", "down", "front", "back", "left", "right", "vertical", "horizontal", "top", "bottom", "inside", "outside", "clockwise", "counterclockwise" and the likes, indicate directions or positional relationships based on directions or positional relationships shown in the accompanying drawings, and are only for the convenience of describing this disclosure and simplifying description, rather than indicating or implying that the device or element referred to must have a specific direction, be constructed and operated in a specific direction, and therefore cannot be understood as a limitation on this disclosure. In addition, the terms "first" and "second" are used for descriptive purposes only, and cannot be understood as indicating or implying relative importance or implicitly indicating the quantity of the indicated technical features. Therefore, the features defined as "first" or "second" may explicitly or implicitly include one or more of the features. In the description of this disclosure, "multiple" means two or more than two, unless otherwise clearly and specifically defined.

Some embodiments of this disclosure are described in detail below in combination with the accompanying drawings. The following embodiments and features in the embodiments may be combined with each other without conflict.

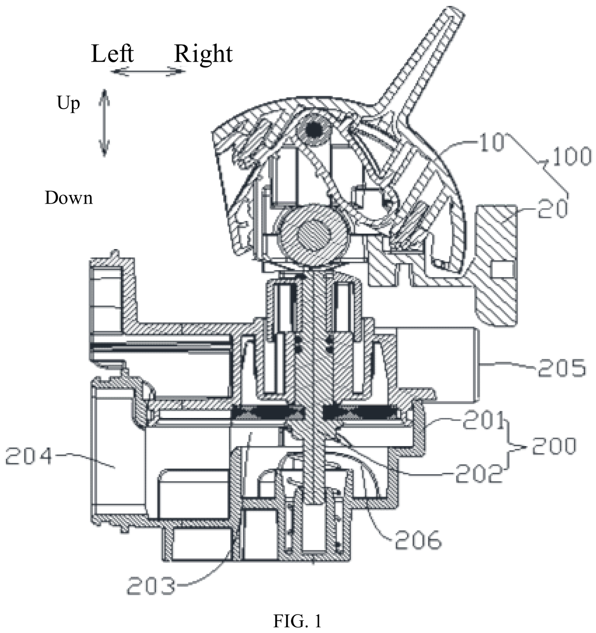

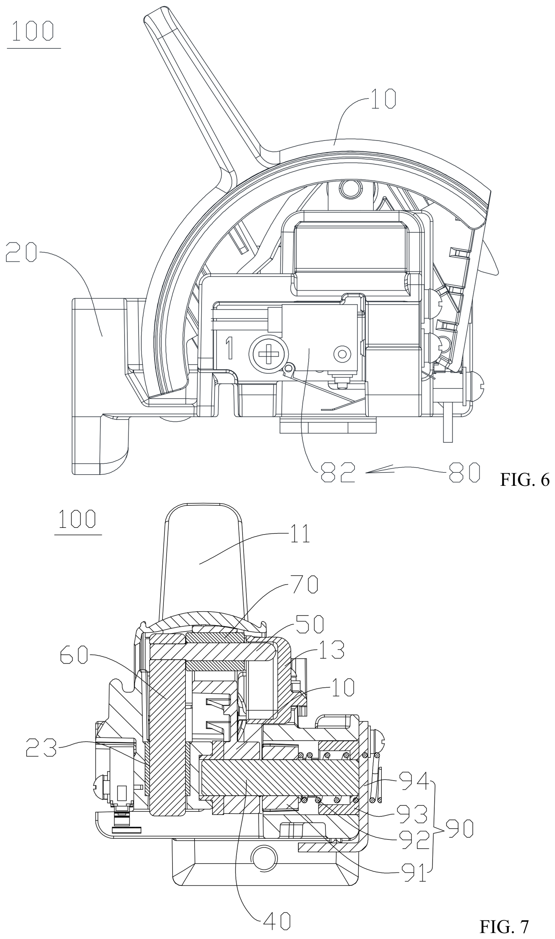

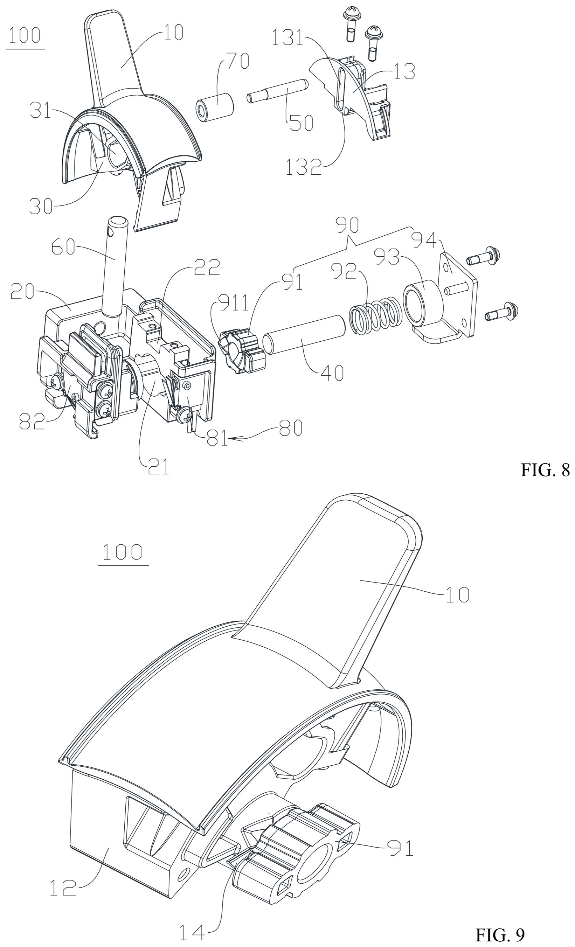

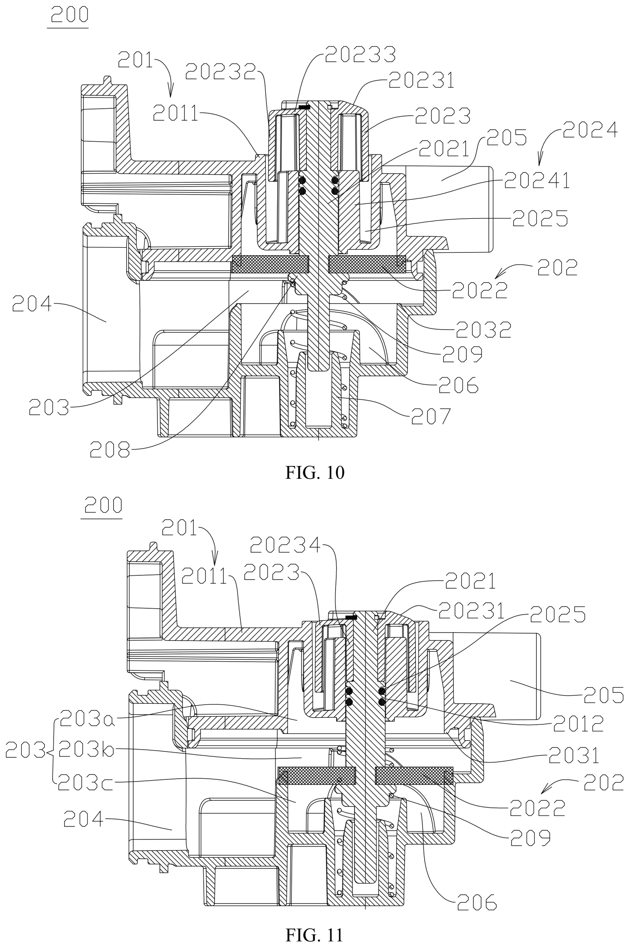

As shown in FIGS. 1 to 12, according to a first aspect of this disclosure, this disclosure provides a switching apparatus, which includes a handle unit 100 and a valve unit 200, wherein the valve unit includes a valve body 201 and a valve core 202 which is capable of moving relative to the valve body 201, wherein the valve body 201 is provided with a valve cavity 203, a respiratory loop interface 204, a machine-controlled branch interface 205 and a manual branch interface 206; wherein the machine-controlled branch interface 205 and the manual branch interface 206 are connected with the respiratory loop interface 204 through the valve cavity 203, the respiratory loop interface 204 is connected with a respiratory loop, the machine-controlled branch interface 205 is connected with a machine-controlled branch, the manual branch interface 206 is connected with a manual branch, the handle unit 100 is configured to control the valve core 202 to switch a ventilation mode of the valve unit 200.

Exemplarily, when the valve unit 200 is in a machine-controlled ventilation mode, the respiratory loop interface 204 is connected with the machine-controlled branch interface 205; when the valve unit 200 is in a manual ventilation mode, the respiratory loop interface 204 is connected with the manual branch interface 206.

Specifically, the handle unit 100 is configured to drive the valve core 202 to move relative to the valve body 201, so as to enable the valve core 202 to control the valve cavity 203 to connect the respiratory loop interface 204 and the machine-controlled branch interface 205, or enable the valve cavity 203 to connect the respiratory loop interface 204 and the manual branch interface 206.

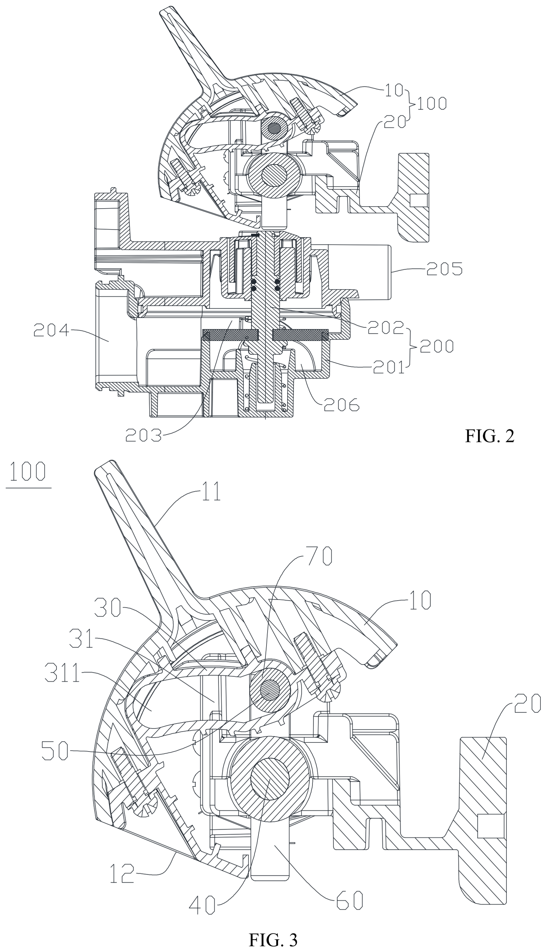

In an optional embodiment, as shown in FIG. 1 and FIG. 2, the handle unit 100 includes a handle body 10 and a fixed support 20, wherein the handle body 10 is rotatably connected with the support 20 for switching the ventilation mode of the valve unit 200. Wherein, the handle body 10 is capable of rotating from a first state to a second state, so as to drive the valve unit 200 to switch from a first mode to a second mode. When the valve unit 200 is in the first mode, the valve core 202 inside the valve cavity 203 controls the respiratory loop interface 204 to be connected with the manual branch interface 206, that is, the valve unit 200 is in a manual ventilation mode; when the valve unit 200 is in the second mode, the valve core 202 inside the valve cavity 203 controls the respiratory loop interface 204 to be connected with the machine-controlled branch interface 205, that is, the valve unit 200 is in a machine-controlled ventilation mode.

This description takes an example in which the first mode is the manual ventilation mode and the second mode is the machine-controlled ventilation mode, but this disclosure is not limited to this. It can also be that when the handle body 10 is in the first state, the valve unit 200 is in the first mode, and the first mode is the machine-controlled ventilation mode; when the handle body 10 is in the second state, the valve unit 200 is in the second mode, and the second mode is the manual ventilation mode.

Exemplarily, when the switching apparatus is assembled at the ventilation device, the valve unit 200 needs to be switched between two different ventilation modes through the handle unit 100. The ventilation device can be, for example, an anesthesia machine or a ventilator, in which the manual ventilation mode is used in an induction stage at a beginning of an operation and a resuscitation stage after an operation, and gas is supplied to a patient by manually squeezing a bag, while the machine-controlled mode is mainly used during an operation, and a machine-controlled ventilation is achieved through a proportional valve or turbine. Therefore, in this disclosure, the handle body 10 is rotated from the first state to the second state or from the second state to the first state, so as to enable the valve unit 200 to be switched between the first mode and the second mode, thereby enabling the ventilation device to be switched between two different ventilation modes.

In an optional embodiment, the handle unit 100 and the valve unit 200 are designed as separate units, so that when a force is applied to the valve unit 200 along a disassembling direction in a first mode or/and a second mode, the valve unit 200 is separated from the handle unit 100; when assembling, a force is applied to the valve unit 200 along an assembling direction which is opposite to the disassembling direction, the valve unit 200 is coupled with the handle unit 100. Wherein, the disassembling direction can be an up-down direction, or a left-right direction, or a lower-left direction, or a lower-right direction as shown in FIG. 2. That is, the handle unit 100 is capable of being connected with or separated from the valve unit 200 along the above direction when the valve unit 200 is in at least one of the machine-controlled ventilation mode and/or the manual ventilation mode. Since the handle unit 100 does not come into contact with a respiratory gas or an anesthesia gas in the respiratory loop interface 204, the machine-controlled branch interface 205 and the manual branch interface 206, there is no need to sterilize the handle unit 100. This disclosure can disassemble the respiratory loop together with the valve unit 200 from the ventilation device for sterilization without disassembling the handle unit 100.

After adopting the above technical solution, since the handle unit 100 and the valve unit 200 are designed as separate units and then assembled with each other, it is not only convenient for a maintenance of the switching apparatus, but also enables that when the handle unit 100 or valve unit 200 malfunctions, only the malfunctioned handle unit 100 or the malfunctioned valve unit 200 needs to be replaced.

The handle unit 100 is isolated from a respiratory gas or an anesthetic gas of a patient and can be separated from the valve unit 200, so it does not need to be sterilized. This not only reduces a labor intensity of medical personnel, but also ensures a sterilization effect of the valve unit 200. At the same time, different manufacturing materials can be selected for the handle unit 100, instead of just manufacturing material of the valve unit 200, which material needs to satisfy requirements of high-temperature sterilization and contacting with the respiratory gas or the anesthetic gas, can be selected, thereby reducing a manufacture cost of the handle unit 100.

In an optional embodiment, when the handle body 10 is in the first state, a first angle is formed between the operation portion 11 of the handle body 10 and a horizontal plane where the support 20 is located (as shown in FIG. 1); when the handle body 10 is rotated from the first state to the second state, a second angle is formed between the operation portion 11 of the handle body 10 and the horizontal plane where the support 20 is located (as shown in FIG. 2), wherein a degree value of the second angle is different from a degree value of the first angle. That is, when the handle body 10 is in the first state, the valve core 202 controls the respiratory loop interface 204 to be connected with the manual branch interface 206, and the valve unit 200 is in the manual ventilation mode now; when the handle body 10 is in the second state, the valve core 202 controls the respiratory loop interface 204 to be connected with the machine-controlled branch interface 205, and the valve unit 200 is in the machine-controlled ventilation mode now, which maintains an operation mode of the existing switching apparatus, is convenient for medical personnel to use according to their usual operation habits, and is also in line with operation habits of medical personnel.

In an optional embodiment, a first elastic member 209 is arranged between the valve core 202 and the valve body 201, and the handle unit 100 abuts against the valve core 202, so as to enable the handle unit 100 and the first elastic member 209 to form a pair of opposite forces along a first direction, thereby enabling the handle body 10 to be stabilized in the first state or the second state relative to the support 20.

Exemplarily, the handle unit 100 and the first elastic member 209 apply opposite forces to the valve core 202, wherein the handle unit 100 applies a downward pressure to the valve core 202, and the first elastic member 209 applies an upward elastic force on the valve core 202, wherein the elastic force and the pressure are collinear opposite forces. When the handle body 10 is in the first state or the second state, the elastic force is equal to the pressure, that is, the handle body 10 is in a stable state relative to the support 20, and correspondingly the valve unit 200 is in the first mode or the second mode. In an optional embodiment, the handle unit 100 includes a handle body 10 and an output member which is capable of moving along a first direction to drive a valve core of the valve unit 200, wherein the output member fits with one end of the valve core 202, and the first elastic member 209 is arranged between the valve body 201 and the other end of the valve core 202. When at the first position and the second position, the handle unit 100 applies a force on the valve core 202 and a direction of said force is opposite to a direction of a force which is applied by the first elastic member 209 on the valve core 202.

Exemplarily, the output member is in transmission connection with the handle body 10, abuts against the valve core 202 and moves between a first position and a second position while the handle body rotates, so as to drive the valve unit 200 to switch between the first mode and the second mode, wherein the first position and the second position have a height difference along the first direction, wherein the first direction is an up-down direction in the drawing; wherein the output member is located outside the valve cavity 203 while the output member moves from the first position to the second position or from the second position to the first position, so as to enable the valve unit 200 to apply a force on the valve unit 200 along a disassembling direction in at least one of the first mode and the second mode, so as to separate the valve unit 200 from the handle unit 100. More preferably, the output member is located outside the valve cavity 203 or the valve body 201 while the output member moves from the first position to the second position.

It should be noted that a movement between the first position and the second position in this description means that the output member moves from the first position to the second position or from the second position to the first position. Moreover, this does not mean that a position of the output member is always between the first position and the second position during the movement. For example, while the output member moves from the first position to the second position, the output member may move to a position beyond the second position and then move back to the second position, vice versa.

It should also be noted that the valve core 202 is assembled inside the valve cavity 203, and a valve rod 2021 of the valve core 202 extends from an inner side of the valve cavity 203, and the output member abuts against one end of the valve rod 2021, which end extends from the valve cavity 203, so as to drive the valve rod 2021 to bring a diaphragm 2022 at the valve rod 2021 to move. Preferably, when the valve unit 200 is in the first mode or the second mode, the output member abuts against one end of the valve rod 2021 which end extends out of the valve cavity 203. For example, when a disassembling direction is a left-right direction shown in FIG. 2, the output member is prevented from extending into the valve cavity 203 so that the valve unit 200 cannot be separated from the handle unit 100; or when the valve unit 200 is in one of the first mode or the second mode, the valve rod 2021 is located at an inner side of the valve cavity 203. At this time, the output member needs to extend into the valve cavity 203 to abut against the valve rod 2021. Therefore, the valve unit 200 cannot be separated from the handle unit 100 in this mode. If a separation is required, the valve unit 200 is adjusted to another mode, so as to enable the valve rod 2021 to extend out of the valve cavity 203, thereby ensuring that the output member is located outside the valve cavity 203.

Exemplarily, when the handle body is in the first state, the output member is at the first position, and the handle body is in a stable state relative to the support 20, that is, a downward pressure applied by the handle unit 100 on the valve core 202 is equal to an upward elastic force applied by the first elastic member 209 on the valve core 202. Similarly, when the handle body is in the second state, the output member is at the second position, and the handle body is also in a stable state relative to the support 20. A downward pressure applied by the handle unit 100 on the valve core 202 is equal to an upward elastic force applied by the first elastic member 209 on the valve core 202.

In an optional embodiment, as shown in FIG. 2, and FIG. 9 to FIG. 12, the valve body 201 is provided with an abutting surface 2011, and the valve core 202 is assembled inside the valve body 201 and extends from the abutting surface 2011.

Exemplarily, the valve core 202 is assembled inside the valve cavity 203 and extends from the abutting surface 2011 at least in the first mode or the second mode in which mode is capable of being disassembled, so that in the first mode or the second mode capable of being disassembled, the valve unit 200 is capable of being pulled along a left direction shown in the drawing to disassemble the valve unit 200 together with a respiratory module. The valve core 202 extending out from the abutting surface 2011 in the first mode or the second mode capable of being disassembled, is capable of avoiding damage to the output member while being disassembled. In other embodiments in which the structure of the illustrated valve body 201 is changed and damage to the output member can also be avoided, the valve unit 200 may be pulled along a right direction to disassemble the valve unit 200 together with the respiratory module. However, this disclosure is not limited thereto, and in the first mode or the second mode capable of being dissembled, the valve unit 200 can be pulled in a downward direction as shown in the drawing to disassemble the valve unit 200 together with the respiratory module. At this time, it is not required that the valve core 202 extends from the abutting surface 2011 in the first mode or the second mode capable of being dissembled.

In an optional embodiment, at least one of the valve core 202 and the handle unit 100 is arranged with a guide structure, which guides the valve unit while assembling the valve unit 100 to the handle unit 100, so that the operator can quickly assemble the valve unit 200 and the handle unit 100 together.

It should be noted that the valve unit 200 can be assembled with the handle unit 100 from a left-right direction, and the valve unit 200 can also be assembled with the handle unit 100 from an up-down direction. Wherein, when the valve unit 200 is assembled with the handle unit 100 from the up-down direction, a guide structure can be arranged at both of the valve core 202 and the handle unit 100, or no guide structure may be arranged at the valve core 202 and the handle unit 100. The guide structure is mainly used to prevent the valve core 202 from being damaged by collision when it is assembled to the handle unit 100. The valve unit 200 can also be assembled with the handle unit 100 from the left-right direction, and a guide structure needs to be arranged at the valve core 202 and the handle unit 100, so as to guide the valve core 202 to be quickly assembled at the handle unit 100.

In an optional embodiment, the guide structure includes a first guide structure and a second guide structure, the first guide structure is arranged at the valve core 202, and the second guide structure is arranged at the handle unit. The first guide structure fits with the second guide structure so that the operator can quickly assemble the valve unit 200 and the handle unit 100 together.

The second guide structure is arranged at the handle body and is capable of rotating along with a rotation of the handle body, so that the valve unit 200 can be assembled with the handle body when the handle body is in any state.

In an optional embodiment, the first guide structure is a first inclined guide surface 20231 arranged at the valve core 202, and the second guide structure is a second inclined guide surface 12 arranged at the handle unit 100, so that with a cooperation of the first inclined guide surface 20231 and the second inclined guide surface 12, the valve unit 200 can be assembled with the handle unit 100 at any assembling angle, thereby improving convenience of assembling the valve unit 200.

When the valve unit 200 and the handle unit 100 are assembled with each other, the valve unit 200 can be connected with the handle unit 100 under a guidance of the first inclined guide surface 20231, so that the ventilation mode of the ventilation device can be switched by rotating the handle body 10, that is, after the handle body 10 is rotated from the first state to the second state, the ventilation device is switched from the manual ventilation mode to the machine-controlled ventilation mode, which meanwhile facilitates disassembling and assembling between the valve unit 200 and the handle unit 100, and enables an operator to squeeze the handle unit 100 without applying too much torque under an action of the first inclined guide surface 20231 and the second inclined guide surface 12, that is, the assembling is convenient and manpower is saved.

Exemplarily, while assembling the valve unit 200, when the valve core 202 contacts the second inclined guide surface 12, or when the first inclined guide surface 20231 contacts the second inclined guide surface 12, since the first inclined guide surface 20231 or the second inclined guide surface 12 has a height difference, that is, when the valve unit 200 is assembled, an end face of the valve core 202 or the first inclined guide surface 20231 will give an upward supporting force on the second inclined guide surface 12. As the valve unit 200 continues to move forward, the second inclined guide surface 12 presses the valve core 202, enables the valve rod 2021 of the valve core 202 to retract into the valve cavity 203, so as to press the first elastic member 209, thereby preventing the valve rod 2021 from being damaged while assembling.

It should be noted that the first guide structure can also be a roller or arc structure which is arranged at the valve core 202, etc., and this disclosure is not limited thereto. Its main purpose is to guide the valve unit 200 to be connected with the handle unit 100 in any mode.

In addition, the second inclined guide surface 12 may not be designed at the handle body 10, and it may be designed as another movement mechanism, which indirectly forms a second guide structure through rotating the handle body 10, and this disclosure does not limit it.

In an optional embodiment, the valve core 202 includes a guide seat 2023 and a valve rod 2021, the guide seat 2023 is assembled at one end of the valve rod 2021 which end is adjacent to the handle unit 100, the first inclined guide surface 20231 is arranged at the guide seat 2023, and the valve rod 2021 is connected with a diaphragm 2022 of the valve core 202, so as to drive the diaphragm 2022 to control respective connection and disconnection between the respiratory loop interface 204 and the machine-controlled branch interface 205, and between the respiratory loop interface 204 and the manual branch interface 206.

Exemplarily, a recessed portion 2025 is formed at a position of the valve body 201 from which position the valve rod 2021 of the valve body 201 protrudes from the valve body 201. The recessed portion 2025 may be formed by being recessed from the abutting surface 2011. The recessed portion 2025 is configured to accommodate the guide seat 2023, and its shape and size are adapted to a shape and size of the guide seat 2023, so that the guide seat 2023 can move along a depth direction of the recessed portion 2025 (i.e., the first direction), so as to drive the diaphragm 2022 connected with the valve rod 2021 to move up and down for controlling respective connection and disconnection between the respiratory loop interface 204 and the machine-controlled branch interface 205, and between the respiratory loop interface 204 and the manual branch interface 206.

In this embodiment, a connection hole for valve rod is arranged at the guide seat 2023, and the valve rod 2021 penetrates into the connection hole for valve rod and fixed by mean(s) of interference fit or fastener(s), such as a retaining ring. The design of the guide seat 2023 and the valve rod 2021 as separate units can facilitate manufacture and assembly of the valve core 202, and can also increase a guide width of the first inclined guide surface 20231. It should be noted that the guide seat 2023 can also be integrally formed with the valve rod 2021. When assembling the valve unit 200, an end of the valve rod 2021, which end is away from the guide seat 2023, extends into the valve cavity 203, and then the diaphragm 2022 can be connected with a side of the valve rod 2021, which side extends into the valve cavity 203. Alternatively, the first inclined guide surface 20231 is arranged at the valve rod 2021 without additionally arranging a guide seat 2023, and this disclosure does not make any limitation thereto.

Except for providing the first inclined guide surface 20231, a ball or a roller may be arranged at the guide seat 2023 to replace the first inclined guide surface 20231. In the case where no guide seat 2023 is provided, a ball or a roller may be arranged at the valve rod 2021 to replace the first inclined guide surface 20231.

In an optional implementation, a first through-hole 2012 extends along a first direction is arranged at the valve body 201, wherein the first through-hole 2012 penetrates through the abutting surface 2011. In the embodiment where the guide seat 2023 is provided, the first through-hole 2012 penetrates through the recessed portion 2025. Specifically, a cylindrical first assembling portion 2024, which extends toward the handle unit 100 along the first direction, is arranged inside the recessed portion 2025, and the first through-hole 2012 penetrates through the cylindrical first assembling portion. In other words, the first assembling portion 2024 surrounds an outer side of the first through-hole 2012. The guide seat 2023 is assembled at the first assembling portion and can move along the first direction. The valve rod 2021 penetrates through and is arranged inside the first through-hole 2012 and moves along the first direction, so as to drive the guide seat 2023 to move outside of the first assembling portion along the first direction. In the first mode or the second mode, the guide seat 2023 is completely located inside the recessed portion 2025.

Specifically, the guide seat 2023 includes an end cover portion 20233, and a first fitting portion 20232 which extends from a periphery of the end cover portion 20233 toward the valve cavity 203 along the first direction; wherein an inner peripheral surface of the first fitting portion 20232 fits with an outer peripheral surface of the first assembling portion 2024 inside the recessed portion 2025, and/or an outer peripheral surface of the first fitting portion 20232 fits with an inner peripheral surface of the recessed portion 2025. A guide structure may be arranged between the first fitting portion 20232 and the first assembling portion 2024/the recessed portion 2025. For example, a guide groove extending along the first direction is arranged at one of the inner peripheral surface of the first fitting portion 20232 and the outer peripheral surface of the first assembling portion 2024, and a guide rib 20241 extending along the first direction is arranged at the other one of the inner peripheral surface of the first fitting portion 20232 and the outer peripheral surface of the first assembling portion 2024. The guide rib 20241 fits with the guide groove to guide a movement of the guide seat 2023.

The first through-hole 2012 is arranged at the guide seat 2023 and extends along the first direction. The guide seat 2023 further includes a second fitting portion 20234 which surrounds the first through-hole 2012 and extends along the first direction. The valve rod 2021 is fixed inside the first through-hole 2012 of the guide seat 2023. The inner peripheral surface of the second fitting portion 20234 is tightly fitted with the valve rod 2021, and the outer peripheral surface of the first assembling portion 2024 is fitted with the first through-hole 2012 at the valve body and is capable of moving along the first through-hole 2012. After adopting the above technical solution, when the switching apparatus is assembled inside the ventilation device, since the handle unit 100 does not need to be sterilized, the handle unit 100 can be fixed at a workbench of the ventilation device through the support 20, and the valve unit 200 needs to be disassembled together with the respiratory loop for sterilization, so it can be snap-fitted into the respiratory loop and connected with the respiratory loop. After the valve unit 200 and the respiratory loop are sterilized, they need to be reassembled with the workbench of the ventilation device. The valve unit 200 can be assembled with the handle unit 100 in cooperation with the first guide structure and the second guide structure, so that the handle body 10 is capable of driving the valve core 202 inside the valve cavity 203 to control the connection and disconnection between the respiratory loop interface 204 and the manual branch interface 206 or between the respiratory loop interface 204 and the machine-controlled branch interface 205, thereby switching the ventilation device between the machine-controlled ventilation mode and the manual ventilation mode, that is, the first guide structure and the second guide structure can cooperate with each other, so as to realize the assembling or disassembling of the valve unit 200 and the handle unit 100 in any ventilation mode.

In an optional embodiment, the valve core 202 includes a first sealing member 2025, which is arranged between an inner wall of the first through-hole and the valve rod 2021, and is configured to seal a gap between the valve rod 2021 and the first through-hole.

In an optional embodiment, the valve core 202 includes a diaphragm 2022, which is fixed at the valve rod 2021 and is capable of moving along the first direction with the valve rod 2021, so as to connect the respiratory loop interface 204 and the machine-controlled branch interface 205, or to connect the respiratory loop interface 204 and the manual branch interface 206.

Exemplarily, an assembling portion for diaphragm is fixedly arranged at the valve rod 2021, and the diaphragm 2022 is assembled at the assembling portion for diaphragm and is capable of moving along the first direction with the valve rod 2021, so as to connect the respiratory loop interface 204 and the machine-controlled branch interface 205, or so as to connect the respiratory loop interface 204 and the manual branch interface 206, thereby controlling the ventilation device to switch between the machine-controlled ventilation mode and the manual ventilation mode. In an optional embodiment, one end of the first elastic member 209 abuts against the valve body 201, and the other end of the first elastic member 209 abuts against the valve rod 2021, so as to apply an elastic force toward the handle unit 100 on the valve rod 2021 for controlling the diaphragm 2022 to close an upper end portion of the valve cavity 203, so as to disconnect the respiratory loop interface 204 from the machine-controlled branch interface 205, while connect the respiratory loop interface 204 with the manual branch interface 206. That is, when the handle unit 100 does not applies a force, the diaphragm 2022 always maintains a state of closing the machine-controlled branch interface 205 under an elastic force of the first elastic member 209.

Exemplarily, the valve cavity 203 is provided with a first cavity body 203a, a second cavity body 203b and a third cavity body 203c; wherein the respiratory loop interface 204 is connected with the second cavity body 203b; the first cavity body 203a is arranged at an upper end of the second cavity body 203b and a first flange 2031 extends from an outer side of an edge of the first cavity body 203a toward the second cavity body 203b; when the diaphragm 2022 is pressed tightly against the first flange 2031 under the elastic force of the first elastic member 209, the diaphragm 2022 will deform when being pressed against the first flange 2031, so as to close the first cavity body 203a for controlling the connection and disconnection between the respiratory loop interface 204 and the machine-controlled branch interface 205. Similarly, a third cavity body 203c is arranged at a lower end of the second cavity body 203b and a second flange 2032 extends from an outer side of an edge of the third cavity body toward the second cavity body 203b. When the diaphragm 2022 moves downward under a drive of the handle unit 100, the deformation of the diaphragm 2022 recovers. When the handle unit 100 is in the first state, the diaphragm 2022 tightly abuts against the second flange 2032 to close the third cavity body, thereby controlling the connection and disconnection between the respiratory loop interface 204 and the manual branch interface 206.

It should be noted that the diaphragm 2022 can be made of a rigid material, which will not be deformed when being pressed against the first flange or the second flange; or, a sealing ring is arranged at the first flange or the second flange, so that when the diaphragm 2022 is pressed against the first flange or the second flange, the sealing ring can be deformed, which can reduce the deformation of the diaphragm 2022 and avoid the possibility that the diaphragm 2022 cannot recover from the deformation after being pressed; or, the diaphragm 2022 is made of an elastic diaphragm with different hardness and thickness at different positions, which is not limited in this disclosure.

Wherein, the diaphragm 2022 can be deformed when being pressed against the first flange 2031, so as to prevent the diaphragm 2022 from moving upward, or the diaphragm 2022 is deformed when being pressed against the first flange 2031, but such deformation does not prevent the diaphragm 2022 from moving upward, and the diaphragm 2022 continues to move upward under a control of the handle unit 100, and this disclosure does not limit this.

Exemplarily, when it is necessary to open the machine-controlled branch interface 205 and close the manual branch interface 206, it is necessary to control through the handle body 10. When the handle body 10 is in the first state, the diaphragm 2022 disconnects the connection between the respiratory loop interface 204 and the machine-controlled branch interface 205 under the elastic force of the first elastic member 209, and the respiratory loop interface 204 is connected with the manual branch interface 206, and the ventilation device is in the manual ventilation mode. When the handle body 10 is in the second state, the diaphragm 2022 disconnects the connection between the respiratory loop interface 204 and the manual branch interface 206 under the drive of the handle body 10, and the respiratory loop interface 204 is connected with the machine-controlled branch interface 205, and the ventilation device is in the machine-controlled ventilation mode.

Exemplarily, a connection portion of valve rod 207 is arranged at the valve body 201, and the connection portion of valve rod 207 is located at a bottom of the valve cavity 203. One end of the valve rod 2021 is connected with the guide seat 2023, and the other end of the valve rod 2021 extends into an interior of the valve cavity 203 through the first through-hole and can move upward and downward along the connection portion of valve rod 207, thereby guiding a movement of the valve rod 2021. Wherein, the assembling portion for diaphragm 208 is arranged at a middle of the valve rod 2021 and is located inside the valve cavity 203, one end of the first elastic member 209 abuts against the valve body 201 and the first elastic body 209 is sleeved outside the connection portion of valve rod 207, and the other end of the first elastic member 209 abuts against the assembling portion for diaphragm 208, so as to enable the diaphragm 2022 assembled at the assembling portion for diaphragm 208 to disconnect the connection between the respiratory loop interface 204 and the machine-controlled branch interface 205; and an external force is required to press the valve rod 2021, so as to enable the valve rod 2021 to move downward along the connection portion of valve rod 207 and press the first elastic member 209, so as to remove a closing of the machine-controlled branch interface 205.

In an optional embodiment, as shown in FIGS. 1 to 9, a guide groove 31 which fits with the output member is arranged at the handle body, and the output member is capable of moving from the first position 311 to the second position 312 along the guide groove 31 while the handle body 10 rotates, so as to drive the valve unit 200 to be switched from the first mode to the second mode. The guide groove 31 applies a force on the output member in a direction which is opposite to a direction in which the first elastic member 209 applies a force on the valve core 202.

Exemplarily, as shown in FIGS. 1 to 4, the output member is provided with a driving member 60 and a fitting member for guide groove 50, wherein the fitting member for guide groove 50 penetrates into the guide groove 31, one end of the driving member 60 is connected with the fitting member for guide groove 50, and the other end of the driving member 60 abuts against the valve unit 200, so as to drive the valve core 202 to control the respiratory loop interface 204 to connect with the machine-controlled branch interface 205 or the manual branch interface 206.

Exemplarily, when the handle body 10 is operated to the first state, the fitting member for guide groove 50 is at the first position 311, and the valve unit 200 is in the first mode now, that is, the respiratory loop interface 204 is connected with the manual branch interface 206; when the handle body is operated to the second state, the fitting member for guide groove 50 is at the second position 312, and the valve unit 200 is in the second mode now, that is, the respiratory loop interface 204 is connected with the machine-controlled branch interface 205.

Wherein, the output member may also only include the driving member 60, one end of the driving member 60 fits with an outer surface of the guide groove 31, or the driving member 60 abuts against an outer surface of the guide groove under an action of an elastic force. At this time, a shape of the guide groove 31 may change and only include a lower half shown in the drawing. When the handle body is operated from the first state to the second state, the driving member 60 is capable of moving from the first position to the second position along the lower end of the guide groove 31, so as to drive the valve unit 200 to be switched from the first mode to the second mode.

In an optional embodiment, the guide groove 31 is a cam surface arranged at the handle body. When at the first position 311 and the second position 312, the output member fits with two ends of the cam surface respectively, so that after the handle body is switched from the first state to the second state, the output member is capable of moving from the first position 311 to the second position 312 along the cam surface, so as to enable the output member to ascend and descend by a height difference of the cam surface along the first direction, and thus pressing the valve rod 2021 or disassembling a pressing on the driving member 60. The valve rod 2021 provides an upward supporting force on the output member under the elastic force of the first elastic member 209, so as to support the handle body 10 and enable the handle body 10 to be stable in the first state or the second state for achieving a bistable state. When switching between the first state or the second state, the handle body 10 needs to be operated to overcome the elastic force of the first elastic member 209.

In an optional embodiment, the output member includes a driving member 60, one end of the driving member 60 is in transmission connection with the guide groove, and the other end of the driving member 60 is configured to drive the valve unit 200, so as to control an up-down movement of the valve core 202 of the valve unit 200, and enable the valve unit 200 to be switched from the first mode to the second mode under a driving of the driving member 60.

In an optional embodiment, a guide hole 23 which extends along a first direction is arranged at the support 20, and the driving member 60 penetrates through and is arranged inside the guide hole 23, wherein the driving member 60 is capable of moving along the guide hole 23 relative to the support 20, so as to drive the valve core 202 to control the respiratory loop interface 204 to be connected with the machine-controlled branch interface 205 or the manual branch interface 206.

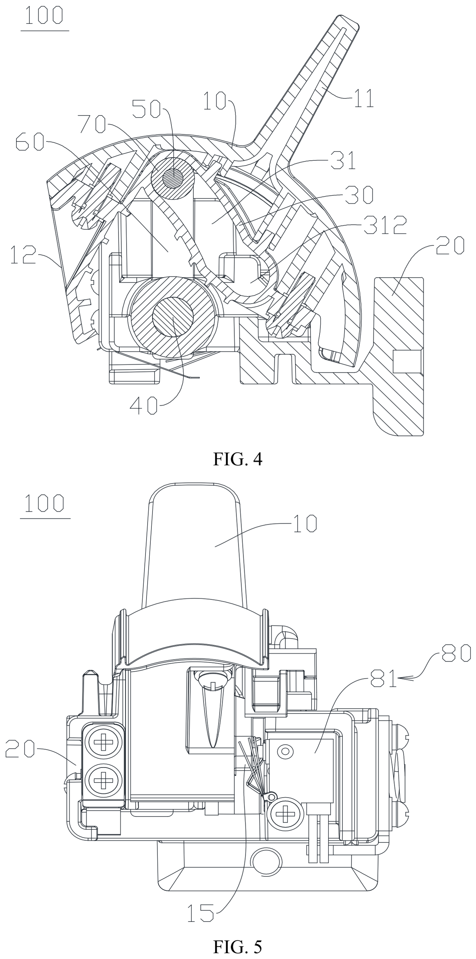

In an optional embodiment, as shown in FIGS. 1 to 4 and 10, when the handle body 10 is in the first state, the valve unit 200 and the handle unit 100 are in a state which is capable of being disassembled (as shown in FIG. 1). In an optional embodiment, as shown in FIGS. 3 and 4, the output member includes a transmission element, and the driving member 60 is movably connected with the guide groove 31 through the transmission element, so as to enable the transmission element to move from the first position 311 to the second position 312along the guide groove 31, thereby driving the driving member 60 to ascend and descend for driving the valve unit 200 to switch the ventilation mode.

In an optional embodiment, the transmission element includes a fitting member for guide groove 50, which is arranged inside the guide groove 31, one end of the fitting member for guide groove 50 is connected with the driving member 60, and the other end of the fitting member for guide groove 50 is arranged inside the anti-rotation groove 131, and the fitting member for guide groove 50 can move from the first position 311 to the second position 312 along the guide groove 31.

In an optional embodiment, the transmission element further includes a rolling member 70, wherein the fitting member for guide groove 50 is movably assembled inside the guide groove 31 via the rolling member 70 to improve a movement smoothness of the fitting member for guide groove 50 inside the guide groove 31. In this embodiment, the rolling element 70 is a cylindrical roller. In another embodiment, the rolling element 70 may also be a rolling bearing, an inner ring of the rolling bearing is connected with the driving element 60, and an outer ring of the rolling bearing is arranged inside the guide groove 31 and can roll along the guide groove 31.

In an optional embodiment, when the output member is at the first position and the second position, the fitting member for guide groove 50 is located at both ends of the guide groove 31 respectively.

Exemplarily, the guide groove 31 is provided with a first end and a second end. When the output member is located at the first position 311, the fitting member for guide groove 50 is at the first end of the guide groove 31. The first end of the guide groove 31 applies a downward pressure on the driving member 60 through the fitting member for guide groove 50, so as to enable the driving member 60 to apply a downward pressure on the valve rod 2021, and an upward elastic force is further applied on the valve rod 2021 by the first elastic member 209, so as to enable the valve rod 2021 and the driving member 60 to abut against each other, that is, the output member and the first elastic member 209 apply opposite forces on the valve core 202 along the first direction, so as to ensure that when the fitting member for guide groove 50 is located at the first end, the switching apparatus is in a stable state.

Similarly, when the output member is located at a second position 312, the fitting member for guide groove 50 is at the second end of the guide groove 31. The second end of the guide groove 31 applies a downward pressure to the driving member 60 through the fitting member for guide groove 50, so as to enable the driving member 60 to apply a downward pressure on the valve rod 2021, and an upward elastic force is further applied on the valve rod 2021 by the first elastic member 209, so as to enable the valve rod 2021 and the driving member 60 to abut against each other, that is, the output member and the first elastic member 209 apply opposite forces on the valve core 202 along the first direction, so as to ensure that when the fitting member for guide groove 50 is located at the second end, the switching apparatus is in a stable state.

It should be noted that when the output member is located at the first position 311, the valve rod 2021 is subjected to an upward elastic force applied by the first elastic member 209, and the diaphragm 2022 and the first flange 2031 are pressed tightly against each other. At this time, the first flange applies a pressure, which is opposite to the elastic force generated by the first elastic member 209, on the valve rod 2021 through the diaphragm 2022. The valve rod 2021 can also be in a stable state under the elastic force of the first elastic member 209 and the pressure of the first flange. When the output member is located at the second position 312, the second end needs to apply a downward pressure on the fitting member for guide groove 50, so as to balance the elastic force applied by the first elastic member 209 on the valve rod 2021.

In an optional embodiment, the fitting member for guide groove 50 is sphere-shaped, wherein an elongated groove is arranged at the guide groove 31 along an extension direction of the guide groove 31, one end of the driving member 60 penetrates through the elongated groove and is connected with the fitting member for guide groove 50, so that when the fitting member for guide groove 50 moves along the guide groove 31 from the first end to the second end, it drives the driving member 60 to move along the first direction. The elongated groove may be arranged at a lower half of the guide groove 31, so as to ensure a balance of the fitting member for guide groove 50 when the guide groove 31 moves.

In an optional embodiment, one end of the driving member 60 is connected with the fitting member for guide groove 50 through a linking member which penetrates through the elongated groove, so as to enable the fitting member for guide groove 50 to drive, via the linking member, the driving member 60 to move along the first direction.

In an optional embodiment, the handle body 10 includes a cam structure 30, a guide groove 31 is arranged inside the cam structure 30, and the cam structure 30 is assembled at the handle body. By designing the cam structure 30 and the handle body as separate units and then assembling them together, a molding process of the handle body and the cam structure 30 can be simplified, thereby reducing a manufacturing cost of the handle body 10.

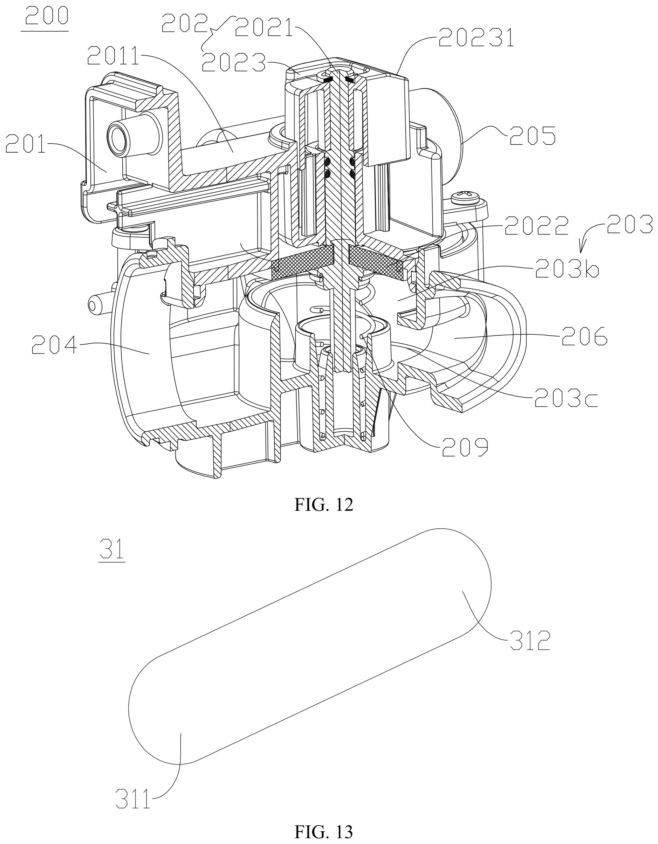

In an optional embodiment, as shown in FIGS. 13 to 18, the guide groove 31 is in a straight-line shape, a bend shape, an arc-curve shape, a V shape, or an inverted V shape, which is not limited in this disclosure. Preferably, the shape of the guide groove 31 enables an inner wall of the guide groove 31 to apply an action force on the fitting member for guide groove 50 of the output member, which force counteracts the elastic force of the first elastic member 209, when the fitting member for guide groove 50 of the output member is located at the first end or the second end of the guide groove 31.

Exemplarily, as shown in FIG. 13, the guide groove 31 is in a straight-line shape, and the guide groove 31 is inclined upward from left to right.

In an optional embodiment, the guide groove 31 includes a first track groove 311a and a second track groove 312a that are interconnected with each other, wherein the first track groove 311a and the second track groove 312a intersect with each other to form an obtuse angle.

In an optional embodiment, at least one of the first track groove 311a and the second track groove 312a is inclined from an intersection of the first track groove 311a and the second track groove 312a toward a direction away from the valve body 201.

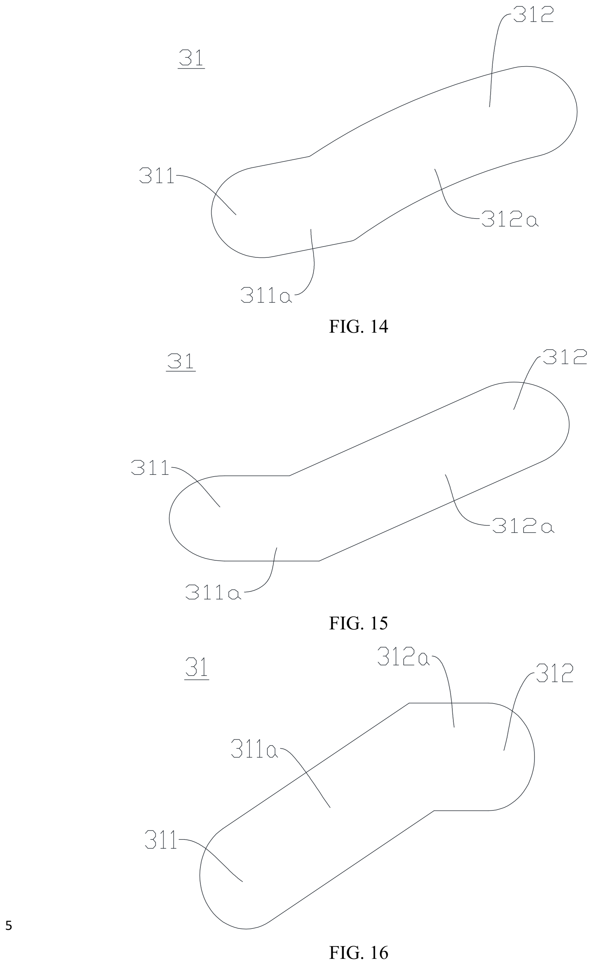

As shown in FIG. 14, the guide groove 31 is in a bend shape, and includes a first track groove 311a and a second track groove 312a which intersect with each other to form an obtuse angle. When the output member is at the first position, the output member is located at a first end 311 of the first track groove 311a, which end is away from the second track groove 312a; when the output member is at the second position, the output member is located at a second end 312 of the second track groove 312a, which end is away from the first track groove 311a, wherein the first track groove 311a and the second track groove 312a can be in a straight-line shape or in an arc shape.

As shown in FIG. 15, the guide groove 31 is in a V shape, and includes a first track groove 311a and a second track groove 312a. An extension line of the first track groove 311a intersects with the second track groove 312a to form an obtuse angle. However, this disclosure is not limited thereto, and a right angle or an acute angle may also be formed.

When the output member is at the first position, the output member is located at a first end 311 of the first track groove 311a, which end is away from the second track groove 312a; when the output member is at the second position, the output member is located at a second end 312 of the second track groove 312a, which end is away from the first track groove 311a, wherein the first track groove 311a and the second track groove 312a can be in a straight-line shape.

As shown in FIG. 16, the guide groove 31 is in an inverted V shape, and includes a first track groove 311a and a second track groove 312a. An extension line of the first track groove 311a intersects with the second track groove 312a to form an obtuse angle. When the output member is at the first position, the output member is located at a first end 311 of the first track groove 311a, which end is away from the second track groove 312a; when the output member is at the second position, the output member is located at a second end 312 of the second track groove 312a, which end is away from the first track groove 311a, and the first track groove 311a and the second track groove 312a can be in a straight-line shape.

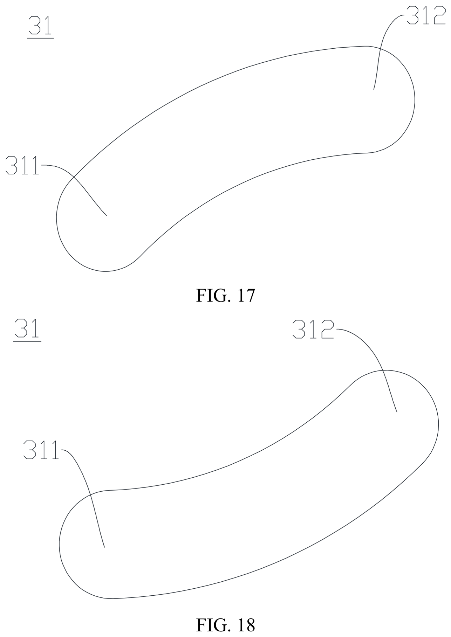

As shown in FIG. 17, the guide groove 31 is in an upward convex-arc shape, and the guide groove 31 is convex upward to form an upward convex-arc structure.

As shown in FIG. 18, the guide groove 31 is in a downward concave-arc shape, and the guide groove 31 is concave downward to form a concave-arc structure.

As shown in FIGS. 3 and 13 to 18, when the handle body 10 rotates around the support 20, the fitting member for guide groove 50 moves along the guide groove 31 from the first end 311 to the second end 312 or from the second end 312 to the first end 311. Since the first position and the second position have a height difference along the first direction, the driving member 60 moves upward and downward when the fitting member for guide groove 50 moves along the guide groove 31, thereby driving the valve core 202 to control the respiratory loop interface 204 to be connected with the machine-controlled branch interface 205 or the manual branch interface 206.

In an optional embodiment, as shown in FIGS. 1 to 9, at least one of the first track groove 311a and the second track groove 312a is inclined along a direction which is away from the valve body 201, so that the fitting member for guide groove 50 can slowly ascend or descend along the guide groove 31, so as to avoid impact caused by ascending or descending too fast. At the same time, when the handle body 10 is in the first state or the second state, the handle body 10 can be arranged at different angles relative to the support 20, so as to facilitate medical personnel to identify and make corresponding switching.

In an optional embodiment, an operation portion 11 is arranged at the handle body, and an extension line of the operation portion 11 and an axis of the driving member 60 form an acute angle relative to each other. This not only saves effort and facilitates medical personnel to switch from the first state to the second state, but also ensures that the fitting member for guide groove 50 can maintain a stable state in the first position 311 and the second position 312 under the action of the first elastic member 209. For example, the fitting member for guide groove 50 is in a first stable state when at the first end 311, and in a second stable state when at the second end 312. The fitting member for guide groove 50 can be transformed from the first stable state to the second stable state, or from the second stable state to the first stable state.

In an optional embodiment, the handle unit 100 includes a rotation shaft 40, and the handle body is rotatably assembled at the support 20 via the rotation shaft 40. A rotation range of the operation portion 11 around the rotation shaft 40 is, for example, within a range of 0 to 90 degrees.

In an optional embodiment, an extended line of the operation portion 11 and an axis of the driving member 60 are arranged to have an angle of ±30 degrees relative to each other.

In an optional embodiment, one end of the fitting member for guide groove 50 is connected with the driving member 60, the fitting member for guide groove 50 is arranged inside the guide groove 31 and fits with the guide groove 31, an anti-rotation groove 131 is arranged at the support 20 along the first direction, and the other end of the fitting member for guide groove 50 is movably connected within the anti-rotation groove 131.

Specifically, the support 20 includes a support body and an anti-rotation frame 13 which is assembled at the support body, wherein the anti-rotation frame 13 is provided with an anti-rotation groove 131 arranged along a first direction, wherein one end of the fitting member for guide groove 50 is movably connected inside the anti-rotation groove 131. When the handle body rotates relative to the support 20, the fitting member for guide groove 50 can move along the first direction under a combined action of the guide groove 31 and the anti-rotation groove 131.

Exemplarily, one end of the fitting member for guide groove 50 is movably connected inside the anti-rotation groove 131, and the other end of the fitting member for guide groove 50 is connected with the driving member 60, so that when the handle body rotates relative to the support 20, the fitting member for guide groove 50 can move along the first direction under a combined action of the guide groove 31 and the anti-rotation groove 131.

In an optional embodiment, a position-limiting platform 132 is arranged at the anti-rotation frame 13, and a position-limiting groove 22 is arranged at the support body. The position-limiting groove 22 is inserted into the position-limiting platform 132 to guide the anti-rotation frame 13 to be assembled at the support body. By utilizing the design of separate units, a mold design structure of the support 20 can be simplified, thereby reducing a manufacturing cost of the support 20.

In an optional embodiment, the handle unit 100 includes a pre-tightened member 90, which is assembled at the support 20 and abuts against the handle body 10, and is configured to prevent the handle body 10 from rotating relative to the support 20 when the handle body 10 is at the first position and the second position, so as to enable the fitting member for guide groove 50 to always maintain a stable state at the first position 311 and the second position 312. This not only allows the handle body 10 to maintain a stable state under the action of pre-tightened member 90 when the valve unit 200 is separated from the handle unit 100, but also prevents the handle body 10 from moving freely between the first position 311 and the second position 312, thereby preventing accidental touch(es). That is, when the handle body 10 is switched between the first state and the second state, a certain external force needs to be applied to the handle body 10 to overcome a resistance applied by pre-tightened member 90 on the handle body 10 while switching.

In an optional embodiment, the pre-tightened member 90 includes a second elastic member 92, and a pre-tightened wheel 91 that fits with the handle body 10, one end of the second elastic member 92 abuts against the support 20, and the other end of the second elastic member 92 abuts against the pre-tightened wheel 91, the pre-tightened wheel 91 abuts against the handle body 10, so as to enable the handle body 10 to be stabilized in the first state or the second state.

Specifically, the support 20 includes a support body and a fixation support frame 94. The support body is provided with a pre-tightened groove 21 whose shape is adapted to that of the pre-tightened wheel 91. The pre-tightened wheel 91 can be movably assembled inside the pre-tightened groove 21, and the second elastic member 92 is arranged between the pre-tightened wheel 91 and the fixation support frame 94, so as to apply a pre-tightened force on the pre-tightened wheel 91 toward the handle body 10.

The pre-tightened member 90 further includes an assembling member for pre-tightened member 93. The second elastic member 92 may be a compression spring. One end of the compression spring is assembled inside the assembling member for pre-tightened member 93. The assembling member for pre-tightened member 93 is connected with a fixation support frame 94.

In an optional embodiment, a protruded structure 911 is arranged at one of the pre-tightened wheel 91 and the handle body, and a recessed structure 14 is arranged at the other one of the pre-tightened wheel 91 and the handle body, wherein the recessed structure 14 has a recessed inclined surface, and the protruded structure 911 is snap-fitted into in the recessed structure 14 under an action of the second elastic member 92 at the first position and the second position. When the handle body rotates around the support 20, the protruded structure 911 abuts against the recessed inclined surface and presses the second elastic member 92 as a height of the recessed inclined surface increases.

Exemplarily, the protruded structure 911 is arranged at the pre-tightened wheel 91, and the recessed structure 14 is arranged at the handle body 10, wherein the recessed structure 14 has two adjacent recessed inclined surfaces, and a vertex is formed between the two adjacent recessed inclined surfaces. The protruded structure 911 may be, for example, a convex tooth, and the recessed structure 14 may be, for example, two adjacent concave teeth. When the handle body 10 rotates around the support 20, the protruded structure 911 abuts against the recessed inclined surface and presses the second elastic member 92 as the height of the recessed inclined surface increases, so that the second elastic member 92 is pressed and the pre-tightened wheel 91 retracts along the pre-tightened member groove 21; when the handle body 10 continues to rotate around the support 20 and passes through the vertex of the protruded structure 911, the protruded structure 911 abuts against the adjacent recessed inclined surface. The second elastic member 92 is reset, so as to enable the protruded structure 911 to be snap-fitted into the recessed structure 14 under an action of the second elastic member 92, and maintain the handle body 10 in a stable state without being affected by external forces. At the same time, an external force is required to rotate the handle body 10, so as to separate the recessed structure 14 with the protruded structure 911.

The positions of the protruded structure 911 and the recessed structure 14 are interchangeable, that is, the protruded structure 911 is arranged at the handle body 10, and the recessed structure is arranged at the pre-tightened wheel 91.

Wherein, when the fitting member for guide groove 50 is at the first end 311 and the second end 312, the protrusion structure 911 is snap-fitted inside the recessed structure 14, which can not only play a role in position-limiting and positioning, preventing the handle body 10 from continuing to rotate or rotating relative to the support 20 without an action of an external force, but also enables the handle unit 100 to maintain a stable state after being separated from the valve unit 200. After the handle unit 100 is assembled with the valve unit 200, the handle unit 100 can also maintain a stable position in the first state and the second state under the action of the first elastic member 209. At the same time, a prompt of being in position can be generated, so that the user can know that the handle body 10 has been rotated in position through a feel produced by a cooperation between the recessed structure 14 and the protruded structure 911, thereby realizing the function of prompting the handle body 10 to be in position while rotating.

For example, when the handle body 10 is rotated in position, the protruded structure 911 is snap-fitted into the recessed structure 14 to make a corresponding sound and produce a corresponding feel, and the user can determine whether the handle body 10 is rotated in position by the feel and sound; or the user can also determine based on the position of the handle body 10 relative to the support 20. The structure is simple and easy to operate.

It should be noted that the structure of the output member is not limited to the above-mentioned embodiment. In addition to a straight-rod shape shown in the above drawings, it can also be in other forms as long as it can abut against the valve rod 2022.

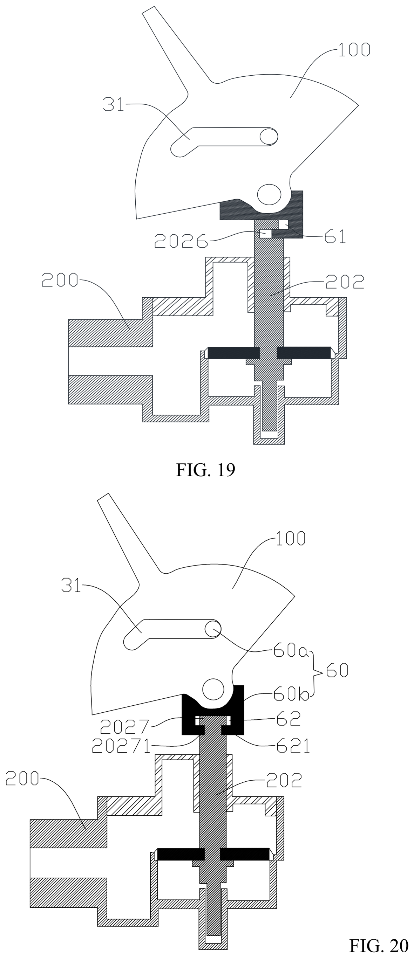

In an optional embodiment, as shown in FIG. 19, a first U-shaped structure 61 is formed at a lower end of the output member, and an opening direction of the first U-shaped structure 61 is toward a disassembling direction of the valve unit 200; correspondingly, a second U-shaped structure 2026 with an opposite opening direction is formed at an upper end of the valve rod 2022, and the first U-shaped structure 61 and the second U-shaped structure 2026 fit with each other, and the first U-shaped structure 61 abuts against the second U-shaped structure 2026 along the first direction.

While disassembling, the valve unit 200 is pulled along the opening direction of the first U-shaped structure 61 at the lower end of the output member, and the valve unit 200 is disassembled together with the respiratory module. The output member only needs to be able to abut against the valve core 202, so as to enable the handle 10 to drive the valve core 202 to move upward and downward, so as to control the respiratory loop interface 204 to be connected with the machine-controlled branch interface 205 or the manual branch interface 206.

For example, when the handle body 10 is in the first state, the valve core 202 closes the machine-controlled branch interface 205 under the drive of the handle body 10 of the first elastic member 209, the respiratory loop interface 204 is connected with the manual branch interface 206, and the ventilation device is in the manual ventilation mode; when the handle body 10 is in the second state, the valve core 202 closes the manual branch interface 206 under the drive of the handle body 10, the respiratory loop interface 204 is connected with the machine-controlled branch interface 205, and the ventilation device is in the machine-controlled ventilation mode.

In an optional embodiment, as shown in FIG. 20, the valve rod 2022 of the valve core 202 is provided with a valve rod head 2027 which extends out of the valve body 201, and the driving member 60 of the output member is provided with a driving section 60a and a connection section 60b. The connection section can be roughly cubic in shape, and a slide way 62 is formed at a bottom of the connection section. The slide way 62 can be formed by, for example, two U-shaped structures arranged opposite to each other, and oppositely arranged protrusions 621 are formed at a bottom of the driving member 60. A slide groove 20271 is formed at the valve rod head 2027 of the valve rod 2022, wherein the slide groove 20271 can be located on both sides of an outer surface of the valve rod head 2027. The slide groove 20271 fits with the protrusion 621. When the driving member 60 moves linearly along the first direction, it can push the valve core 202 to move. When a force is applied toward a direction of a paper surface, the slide groove 20271 can be separated from the protrusion 621, thereby disassembling the valve unit 200.

Exemplarily, one end of the driving section 60a of the output member is connected with the guide groove 31, and the other end of the driving section 60a is connected with the connection section. The valve rod head 2027 fits with the connection section, so that when the handle body 10 is switched between the first state and the second state, the driving section 60a can drive the connection section to move along the first direction, thereby driving, via the valve rod head 2027 connected with the connection section 60b, the valve core 202 to move, so as to switch the ventilation mode of the ventilation device.

In an optional embodiment, a cross-section of the connection section cut perpendicular to the paper surface can be roughly triangular, and a slide way 62 is arranged at a bottom of the triangle, so as to ensure a stability of connection between the driving section and the connection section, while also ensuring that there is enough space in the connection section to open the slide way 62, thereby ensuring a strength of the connection section after the slide way 62 is opened at the connection section.

In an optional embodiment, as shown in FIGS. 1 to 6, the handle unit 100 includes a detection unit 80, which includes a first travel switch 81 which is configured to detect a position of the handle body, and the first travel switch 81 is assembled at the support 20. When the handle body 10 drives the valve core 202 to control the respiratory loop interface 204 to be connected with the manual branch interface 206 or the machine-controlled branch interface 205, the first travel switch 81 is capable of outputting a detection signal to prevent the handle body 10 from not moving in position, thereby ensuring a safety and reliability of switching between the machine-controlled ventilation mode and the manual ventilation mode.

Exemplarily, the first travel switch 81 is provided with a first triggering portion, which extends toward one end of the valve core. The handle body 10 is provided with a triggering member 15, which fits with the first triggering portion to output a detection signal.

Wherein, the first triggering portion can be an elastic sheet or a contact point, and the triggering member 15 can be a protruded structure of the handle body 10 toward the first travel switch 81. While the handle body 10 rotates around the support 20, the protruded structure can abut against the elastic sheet or the contact point, so as to enable the first travel switch 81 to output a detection signal to outside.

In an optional embodiment, the detection unit 80 includes a second travel switch 82 which is configured to detect a position of the valve core of the valve unit, wherein the second travel switch 82 is assembled at the support 20, so as to ensure a safety and reliability of switching between the machine-controlled ventilation mode and the manual ventilation mode.

Exemplarily, the second travel switch 82 is provided with a second triggering portion, which extends toward one end of the valve core 202 so as to enable the valve core 202 to move toward the second triggering portion and trigger the second triggering portion, so as to enable the second travel switch 82 to accurately output signals of the valve core 202 during switching, so as to ensure a correct state.

This disclosure arranges a first travel switch 81 and a second travel switch 82 at the support 20, which are respectively used to detect a rotation position of the handle body 10 and a position of the valve core 202, thereby ensuring that the valve core 202 is in the first mode when the handle body 10 is in the first state, or that the valve core 202 is in the second mode when the handle body 10 is in the second state. As long as one of the first travel switch 81 and the second travel switch 82 does not output a signal to the outside, the system will alarm to remind the user to check whether the machine state is correct. Or when the signals outputted by the first travel switch 81 and the second travel switch 82 are inconsistent with expectations, the switching apparatus is indicated to have not been switched as expected, or the first travel switch 81 and the second travel switch 82 are indicated to malfunction, the system will also alarm to ensure an accuracy of the switching apparatus for switching the ventilation mode.

After adopting the above technical scheme, a state of the handle body 10 and a state of the valve core 202 can be detected at the same time when the handle body 10 is in the first state or the second state, thereby improving the detection accuracy, avoiding the first travel switch 81 and the second travel switch 82 from making erroneous determination due to only one travel switch exists, and improving a safety and reliability of the detection unit 80. Meanwhile, a rapid detection of switching between the machine-controlled ventilation mode and the manual ventilation mode, can be realized, and the operation is simple and convenient.

It should be noted that the detection unit 80 may also be one or more combinations of an infrared tube, a Hall sensor, and a magnetic switch, which is not limited in this disclosure.

As shown in FIGS. 1 to 12, according to a second aspect of this disclosure, a ventilation device is provided, including a main body of ventilation device, a respiratory loop arranged at the main body and the above-mentioned switching apparatus, wherein the switching apparatus is assembled inside the respiratory loop, and the valve unit 200 is connected with the respiratory loop, the handle unit 100 is configured to drive the valve unit 200 to control a ventilation mode of the respiratory loop.

A first snap-fit structure is arranged at the valve unit 200, and a second snap-fit structure is arranged at the main body. When the valve unit 200 is separated from or coupled with the handle unit, the first snap-fit structure is separated from or snap-fitted with the second snap-fit structure accordingly. Preferably, a third guide structure is arranged at the valve unit 200, and a fourth guide structure is arranged at the main body. While separating or coupling the valve unit 200 and the handle unit 100, the third guide structure moves along the fourth guide structure. The third guide structure may be a slide block arranged at the valve unit 200, and the fourth guide structure may be a slide track which is arranged at the ventilation device. The third guide structure and the fourth guide structure may also be in other forms.

The ventilation device may be an anesthesia machine, a ventilator, etc.

In the description of this disclosure, it should be noted that, unless otherwise clearly specified and limited, the terms "assemble", "couple" and "connect" should be understood in a broad sense. For example, the terms can be a fixed connection, a detachable connection, or an integral connection; can be a mechanical connection or an electrical connection; can be a direct connection or an indirect connection through an intermediate medium; can be an internal connection between two elements or an interaction between two elements. For those skilled in the art, the specific meanings of the above terms in this disclosure can be understood according to specific circumstances.

In this disclosure, unless otherwise clearly specified and limited, a first feature being "at" or "on/under" a second feature may include the first and second features being in direct contact, or may include the first and second features not being in direct contact but being indirect contact through another feature between them. Moreover, a first feature being "above", "up" and "upward" relative to a second feature includes the first feature being directly above and diagonally above the second feature, or simply means that the first feature is at a higher level than the second feature. A first feature being "below," "low," and "downward" a second feature includes the first feature being directly below and diagonally below the second feature, or simply means that the first feature is at a lower horizontal height than the second feature.

The above disclosure provides many different embodiments or examples for implementing different structures of this disclosure. In order to simplify the description of this disclosure, components and arrangements of specific examples are described above. Of course, they are merely examples and are not intended to limit this disclosure. In addition, this disclosure may repeat reference numerals and/or reference letters in different examples. Such repetition is for the purpose of simplicity and clarity and does not in itself indicate the relationship between the various embodiments and/or settings discussed. In addition, this disclosure provides examples of various specific processes and materials, but a person skilled in the art may be aware of the application of other processes and/or the use of other materials.

In the description of this description, reference to terms such as "an embodiment", "some embodiments", "illustrative embodiment(s)", "example(s)", "specific example(s)", or "some examples" means that the specific features, structures, materials or characteristics described in conjunction with the embodiment or example are included in at least one embodiment or example of this disclosure. In this description, the schematic expressions of the above terms do not necessarily refer to the same embodiment or example. Furthermore, the specific features, structures, materials, or characteristics described may be combined in any suitable manner in any one or more embodiments or examples.

## Claims

1. A switching apparatus for a ventilation device, **characterized in that**, comprising: a handle unit and a valve unit, wherein the valve unit comprises a valve body and a valve core, wherein the valve body is provided with a valve cavity, a respiratory loop interface, a machine-controlled branch interface and a manual branch interface;
the handle unit comprises a support which is capable of being fixed to the ventilation device, a handle body which is rotatably connected with the support, and an output member which is configured to drive the valve unit; wherein the handle body is capable of rotating relative to the support, the output member abuts against the valve core and moves between a first position and a second position while the handle body rotates, so as to drive the valve core to reciprocate along a first direction, and enable the valve unit to be switched between a first mode and a second mode; wherein in the first mode, the respiratory loop interface is connected with the manual branch interface; in the second mode, the respiratory loop interface is connected with the machine-controlled branch interface;
wherein the handle unit and the valve unit are designed as separate units, and when the support is fixed to the ventilation device and a force is applied to the valve unit in at least one of the first mode or the second mode, the valve unit is separated from or coupled with the handle unit, so as to disassemble or assemble the valve unit.

2. The switching apparatus according to claim 1, **characterized in that**, a first elastic member is arranged between the valve core and the valve body, wherein the output member and the first elastic member apply forces in opposite directions on the valve core along the first direction.

3. The switching apparatus according to claim 1, **characterized in that**, the output member moves along the first direction; the first position and the second position have a height difference along the first direction; the output member is located outside the valve cavity while the output member moves from the first position to the second position.

4. The switching apparatus according to claim 1, **characterized in that**, the valve body is provided with an abutting surface, the valve core is assembled inside the valve cavity and at least extends from the abutting surface in said at least one of the first mode or the second mode.

5. The switching apparatus according to claim 2, **characterized in that**, at least one of the valve core and the handle unit is arranged with a guide structure, which guides the valve unit while assembling the valve unit.

6. The switching apparatus according to claim 5, **characterized in that**, the guide structure comprises a first guide structure and a second guide structure, wherein the first guide structure is arranged at the valve core, the second guide structure is arranged at the handle unit, the first guide structure fits with the second guide structure;
wherein, the first guide structure is a first inclined guide surface arranged at the valve core, and the second guide structure is a second inclined guide surface arranged at the handle unit.

7. The switching apparatus according to claim 4, **characterized in that**, the valve core comprises a guide seat and a valve rod, wherein the guide seat is assembled at one end of the valve rod which end is adjacent to the handle unit.

8. The switching apparatus according to claim 7, **characterized in that**, a recessed portion is arranged at the valve body; a first assembling portion which extends along the first direction and a first through-hole which penetrates through the first assembling portion are arranged inside the recessed portion; wherein the guide seat is assembled at the first assembling portion and is capable of moving along the first direction, the valve rod penetrates through and is arranged inside the first through-hole.

9. The switching apparatus according to claim 8, **characterized in that**, a guide structure is arranged between the guide seat and the recessed portion, or between the guide seat and the first assembling portion.

10. The switching apparatus according to claim 7, **characterized in that**, the valve core comprises a first sealing member, which is arranged between an inner wall of the first through-hole and the valve rod.

11. The switching apparatus according to claim 7, **characterized in that**, the valve core comprises a diaphragm, which is fixed at the valve rod and is capable of moving along the first direction with the valve rod, so as to connect the respiratory loop interface and the machine-controlled branch interface, or so as to connect the respiratory loop interface and the manual branch interface.

12. The switching apparatus according to claim 11, **characterized in that**, the output member comprises a driving member and a fitting member for guide groove, wherein a guide groove is arranged at the handle body, one end of the driving member is in transmission fitting with the guide groove through the fitting member for guide groove, and the other end of the driving member abuts against the valve core.

13. The switching apparatus according to claim 12, **characterized in that**, the fitting member for guide groove is arranged inside the guide groove and fits with the guide groove; wherein one end of the fitting member for guide groove is connected with the driving member; an anti-rotation groove which extends along the first direction is arranged at the support, and the other end of the fitting member for guide groove is movably connected with the anti-rotation groove.

14. The switching apparatus according to claim 12, **characterized in that**, the fitting member for guide groove is sphere-shaped or arc-shaped, and an elongated groove is arranged inside the guide groove along an extension direction of the guide groove; wherein one end of the driving member penetrates through the elongated groove and is connected with the fitting member for guide groove, or one end of the driving member is connected with the fitting member for guide groove through a linking member which penetrates through the elongated groove.

15. The switching apparatus according to claim 14, **characterized in that**, the guide groove comprises a first track groove and a second track groove that are connected with each other, wherein the first track groove intersects with the second track groove to form an obtuse angle.

16. The switching apparatus according to claim 15, **characterized in that**, at least one of the first track groove and the second track groove is inclined from an intersection of the first track groove and the second track groove toward a direction which is away from the valve body.

17. The switching apparatus according to claim 12, **characterized in that**,
one end of the fitting member for guide groove is connected with the driving member, and the fitting member for guide groove is arranged inside the guide groove; or
the output member further comprises a rolling member, one end of the fitting member for guide groove is connected with the driving member, and the fitting member for guide groove is movably assembled inside the guide groove through the rolling member.

18. The switching apparatus according to claim 7, **characterized in that**, a first U-shaped structure is formed at a lower end of the output member, and an opening direction of the first U-shaped structure is toward a disassembling direction of the valve unit; a second U-shaped structure with an opposite opening direction is formed at an upper end of the valve rod, wherein the first U-shaped structure fits with the second U-shaped structure and abuts against the second U-shaped structure along the first direction.

19. The switching apparatus according to claim 3, **characterized in that**, the handle unit further comprises a pre-tightened member which is assembled at the support and abuts against the handle body, so as to prevent the handle body from rotating relative to the support when the output member is located at the first position and the second position.

20. The switching apparatus according to claim 19, **characterized in that**, the pre-tightened member comprises a second elastic member and a pre-tightened wheel which wheel fits with the handle body, wherein one end of the second elastic member abuts against the support, and the other end of the second elastic member abuts against the pre-tightened wheel, so as to enable the pre-tightened wheel to abut against the handle body.

21. The switching apparatus according to claim 20, **characterized in that**, a protruded structure is arranged at one of the pre-tightened wheel and the handle body; and a recessed structure is arranged at the other of the pre-tightened wheel and the handle body, wherein the recessed structure is provided with a recessed inclined surface; when the output member is located at the first position and the second position, the protruded structure is snap-fitted into the recessed structure under an action of the second elastic member; when the handle body rotates around the support, the protruded structure abuts against the recessed inclined surface and presses the second elastic member as a height of the recessed inclined surface increases.

22. The switching apparatus according to claim 12, **characterized in that**, the handle unit further comprises a first travel switch which is configured to detect a position of the handle body, wherein the first travel switch is assembled at the support.

23. The switching apparatus according to claim 22, **characterized in that**, the first travel switch is provided with a first triggering portion, which extends toward one side of the handle body.

24. The switching apparatus according to claim 22, **characterized in that**, the handle unit further comprises a second travel switch which is configured to detect a position of the valve core of the valve unit, wherein the second travel switch is assembled at the support.

25. The switching apparatus according to claim 24, **characterized in that**, the second travel switch is provided with a second triggering portion, which extends toward one end of the valve core.

26. A ventilation device, **characterized in that**, comprising a respiratory loop and the switching apparatus according to any one of claims 1~25; wherein the switching apparatus is assembled inside the respiratory loop, and the valve unit is connected with the respiratory loop.

27. The ventilation device according to claim 26, **characterized in that**, further comprising a main body, a first snap-fit structure which is arranged at the valve unit, and a second snap-fit structure which is arranged at the main body; when the valve unit is separated from or coupled with the handle unit, the first snap-fit structure and the second snap-fit structure are correspondingly separated from or snap-fitted with each other.

28. The ventilation device according to claim 26, **characterized in that**, a third guide structure is arranged at the valve unit, and a fourth guide structure is arranged at the main body; wherein the third guide structure moves along the fourth guide structure, while the valve unit and the handle unit are separated from or coupled with each other.

29. The ventilation device according to claim 26, **characterized in that**, the third guide structure is a slide block which is arranged at the valve unit, and the fourth guide structure is a slide track which is arranged at the ventilation device.
